# EUROPEAN PATENT APPLICATION

(11) **EP 1 852 513 A2**
(43) Date of publication of application: **07.11.2007**
(21) Application number: 07007420.8
(22) Date of filing: 11.04.2007
(51) Int. Cl.: C12Q 1/68

(54) **Method for diagnosing cancer using cancer-associated deletion gene marker**

(30) Priority: 12.04.2006 JP 2006109312
(71) Applicant: FUJIFILM Corporation, Minato-ku Tokyo 106-0031 (JP); Tokyo Medical and Dental University, Tokyo 113-8510 (JP)
(72) Inventor: Inazawa, Johji, Tokyo 113-8510 (JP); Imoto, Issei, Tokyo 113-8510 (JP); Izumi, Hiroyuki, Tokyo 113-8510 (JP); Yokoi, Sana, Tokyo 113-8510 (JP)
(74) Representative: HOFFMANN EITLE

(57) **Abstract**

It is an object of the present invention to provide a novel method for diagnosing cancer by discovering a cancer-associated gene which could not be detected by a conventional technique, and detecting deletion, mutation, or an abnormal expression level of such cancer-associated gene. The present invention pmvides a method for diagnosing cancer which comprises a step of detecting deletion of the GMDS, ANKRD15, TEK, or EBI2 gene in a test sample by using DNA containing all or part of said gene.

SEQUENCE LISTING
<110> Fuji Photo Film Co.
<120> A method for diagnosing cancer using cancer-related deletion gene marker
<130> A61274A
<160> 10
<210> 1
<211> 1698
<212> DNA
<213> Homo sapiens
<400> 1
cccggccctc cctgcacggc ctcccgtgcg cccctgtcag actgtggcgg ccggtcgcgc 60
ggtgcgctct ccctccctgc ccgcagcctg gagaggcgct tcgtgctgca cacccccgcg 120
ttcctgccgg caccgcgcct gccctctgcc gcgctccgcc ctgccgccga ccgcacgccc 180
gccgcgggac atggcacacg caccggcacg ctgccccagc gcccggggct ccggggacgg 240
cgagatgggc aagcccagga acgtggcgct catcaccggt atcacaggcc aggatggttc 300
ctacctggct gagttcctgc tggagaaagg ctatgaggtc catggaattg tacggcggtc 460
cagttcattt aatacgggtc gaattgagca tctgtataag aatccccagg ctcacattga 420
aggaaacatg aagttgcact atggcgatct cactgacagt acctgccttg tgaagatcat 480
taatgaagta aagcccacag agatctacaa ccttggagcc cagagccacg tcaaaatttc 540
ctttgacctc gctgagtaca ctgcggacgt tgacggagtt ggcactctac gacttctaga 600
tgcagttaag acttgtggcc ttatcaactc tgtgaagttc taccaagcct caacaagtga 660
actttatggg aaagtgcagg aaatacccca gaaggagacc acccctttct atccccggtc 720
accctatggg gcagcaaaac tctatgccta ttggattgtg gtgaacttcc gtgaggcgta 780
taatctcttt gcagtgaacg gcattctctt caatcatgag agtcccagaa gaggagctaa 840
tttcgttact cgaaaaatta gccggtcagt agctaagatt taccttggac aactggaatg 900
tttcagtttg ggaaatctgg atgccaaacg agattggggc catgccaagg actatgtgga 960
ggctatgtgg ttgatgttgc agaatgatga gccggaggac ttcgttatag ctactgggga 1020
ggtccatagt gtccgggaat ttgtcgagaa atcattcttg cacattggaa aaaccattgt 1080
gtgggaagga aagaatgaaa atgaagtggg cagatgtaaa gagaccggca aagttcacgt 1140
gactgtggat ctcaagtact accggccaac tgaagtggac tttctgcagg gcgactgcac 1200

caaagcgaaa cagaagctga actggaagcc ccgggtcgct ttcgatgagc tggtgaggga 1260
gatggtgcac gccgacgtgg agctcatgag gacaaacccc aatgcctgag cagcgcctcg 1320
gagcccggcc cgccctccgg ctacaatccc cgcagagtct ccggtgcaga cgcgctgcgg 1380
ggatggggag cggcgtgcca atctgcgggt cccctgcggc ccctgctgcc gctgcgctgt 1440
cccggccgca agagcggggc cgccccgccg aggtttgtag cagccgggat gtgaccctcc 1500
agggtttggg tcgctttgcg tttgtcgaag cctcctctga atggctttgt gaaatcaaga 1560
tgttttaatc acattcactt tacttgaaat tatgttgtta cacaacaaat tgtggggcct 1620
tcaaattgtt tttctctttt catattaaaa atggtctttc tgtgaactag caaaaaaaaa 1680
aaaaaaaaaa aaaaaaaa 1698
<210> 2
<211> 372
<212> PRT
<213> Homo sapiens
<400> 2
Met Ala His Ala Pro Ala Arg Cys Pro Ser Ala Arg Gly Ser Gly Asp
1               5                   10                  15
Gly Glu Met Gly Lys Pro Arg Asn Val Ala Leu Ile Thr Gly Ile Thr
            20                  25                  30
Gly Gln Asp Gly Ser Tyr Leu Ala Glu Phe Leu Leu Glu Lys Gly Tyr
        35                  40                  45
Glu Val His Gly Ile Val Arg Arg Ser Ser Ser Phe Asn Thr Gly Arg
    50                  55                  60
Ile Glu His Leu Tyr Lys Asn Pro Gln Ala His Ile Glu Gly Asn Met
65                  70                  75                  80
Lys Leu His Tyr Gly Asp Leu Thr Asp Ser Thr Cys Leu Val Lys Ile
                85                  90                  95
Ile Asn Glu Val Lys Pro Thr Glu Ile Tyr Asn Leu Gly Ala Gln Ser
            100                 105                 110
His Val Lys Ile Ser Phe Asp Leu Ala Glu Tyr Thr Ala Asp Val Asp
        115                 120                 125

Gly Val Gly Thr Leu Arg Leu Leu Asp Ala Val Lys Thr Cys Gly Leu
    130                 135                 140
Ile Asn Ser Val Lys Phe Tyr Gln Ala Ser Thr Ser Glu Leu Tyr Gly
145                 150                 155                 160
Lys Val Gln Glu Ile Pro Gln Lys Glu Thr Thr Pro Phe Tyr Pro Arg
                165                 170                 175
Ser Pro Tyr Gly Ala Ala Lys Leu Tyr Ala Tyr Trp Ile Val Val Asn
            180                 185                 190
Phe Arg Glu Ala Tyr Asn Leu Phe Ala Val Asn Gly Ile Leu Phe Asn
        195                 200                 205
His Glu Ser Pro Arg Arg Gly Ala Asn Phe Val Thr Arg Lys Ile Ser
    210                 215                 220
Arg Ser Val Ala Lys Ile Tyr Leu Gly Gln Leu Glu Cys Phe Ser Leu
225                 230                 235                 240
Gly Asn Leu Asp Ala Lys Arg Asp Trp Gly His Ala Lys Asp Tyr Val
                245                 250                 255
Glu Ala Met Trp Leu Met Leu Gln Asn Asp Glu Pro Glu Asp Phe Val
            260                 265                 270
Ile Ala Thr Gly Glu Val His Ser Val Arg Glu Phe Val Glu Lys Ser
        275                 280                 285
Phe Leu His Ile Gly Lys Thr Ile Val Trp Glu Gly Lys Asn Glu Asn
    290                 295                 300
Glu Val Gly Arg Cys Lys Glu Thr Gly Lys Val His Val Thr Val Asp
305                 310                 315                 320
Leu Lys Tyr Tyr Arg Pro Thr Glu Val Asp Phe Leu Gln Gly Asp Cys
                325                 330                 335
Thr Lys Ala Lys Gln Lys Leu Asn Trp Lys Pro Arg Val Ala Phe Asp
            340                 345                 350
Glu Leu Val Arg Glu Met Val His Ala Asp Val Glu Leu Met Arg Thr
        355                 360                 365

Asn Pro Asn Ala
    370
<210> 3
<211> 5083
<212> DNA
<213> Homo sapiens
<400> 3
ggtccgcggc ggagcgagcg agcggccggc aggttgggag gagcggccga aggttgaatg 60
cctttgagaa cttgatgcat aaaatttgca tgactcctca ctcctttctg gatctctcat 120
tggactcaag ccagcatggc tcacaccaca aaggttaacg gcagtgcctc aggaaaagca 180
ggtgatattc tcagtggaga ccaggacaag gaacagaaag acccttactt tgtggagacc 240
ccctatggtt atcaactaga cttagatttc ctcaaatatg tggatgacat acagaaggga 300
aataccatca aaagactgaa catccagaag aggcggaagc cgtccgtgcc atgcccagaa 360
cccaggacca catctggtca gcaaggtata tggacttcca ctgaatccct ctcatcctcc 420
aacagtgatg acaacaagca gtgccccaac ttcctcatag ccagaagtca agttacatca 480
actccaatct caaagccacc tccccctctg gagacctcac tcccttttct taccatccca 540
gaaaatcgac agctgccacc tccctcacca caactcccaa agcataacct tcatgtcacc 600
aagacactga tggagacccg gagaagactg gaacaggaga gagccaccat gcagatgaca 660
ccgggtgagt tcagaaggcc caggctggcc agttttggag gcatgggcac cacaagctcc 720
ctcccttctt ttgtgggttc tggaaaccac aatcctgcca agcaccagct tcagaatgga 780
taccaaggta atggggatta tggtagctat gccccagctg ctcccaccac ttcctccatg 840
gggagctcca tccgccacag ccccctgagc tcagggatct ccaccccagt gaccaacgtg 900
agccccatgc acctgcagca catccgcgag cagatggcca ttgctctgaa acgcctgaag 960
gagctggagg agcaggtgcg aaccatccct gtgctccagg taaagatctc tgtcttgcaa 1020
gaagagaaaa ggcagttggt ctcacagctg aaaaaccaaa gggctgcatc ccagatcaat 1080
gtctgtggtg tgaggaagcg gtcctatagt gcggggaacg cctcccagct ggaacagctc 1140
tcccgggccc gaagaagtgg cggggaatta tacattgact atgaggagga agaaatggag 1200
accgtagaac agagcacgca gaggataaag gagttccggc aacttacagc agacatgcaa 1260
gccctggagc agaagatcca ggacagcagc tgtgaggcct cctcagagct cagggagaat 1320
ggagagtgcc ggtctgtggc tgtgggtgcc gaggagaaca tgaacgacat cgtcgtgtac 1380

cacagaggct ccaggtcctg taaggatgca gctgtaggga cacttgttga gatgagaaat 1440
tgtggggtca gcgtgacaga ggccatgctt ggagtgatga ctgaagctga caaagaaatt 1500
gagctgcaac agcagaccat agaatccttg aaggaaaaga tctatcgcct agaagtacag 1560
cttagagaaa ccacccatga ccgggagatg actaaactga aacaagagct gcaggctgct 1620
ggatcgagga aaaaggttga caaagccacg atggcccagc cgcttgtttt cagtaaggtg 1680
gtggaggcag tggtgcagac cagagaccaa atggtcggca gtcacatgga cctggtggac 1740
acgtgtgttg ggacctccgt ggaaacaaac agtgtaggca tctcctgcca gcctgaatgt 1800
aagaataaag tcgtagggcc tgagctgcct atgaattggt ggattgttaa ggagagggtg 1860
gaaatgcatg accgatgtgc tgggaggtct gtggaaatgt gtgacaagag tgtgagtgtg 1920
gaagtcagcg tctgcgaaac aggcagcaac acagaggagt ctgtgaacga cctcacactc 1980
ctcaagacaa acttgaatct caaagaagtg cggtctatcg gttgtggaga ttgttctgtt 2040
gacgtgaccg tctgctctcc aaaggagtgc gcctcccggg gcgtgaacac tgaggctgtt 2100
agccaggtgg aagctgccgt catggcagtg cctcgtactg cagaccagga cactagcaca 2160
gatttggaac aggtgcacca gttcaccaac accgagacgg ccaccctcat agagtcctgc 2220
accaacactt gtctaagcac tttggacaag cagaccagca cccagactgt ggagacgcgg 2280
acagtagctg taggagaagg ccgtgtcaag gacatcaact cctccaccaa gacgcggtcc 2340
attggtgttg gaacgttgct ttctggccat tctgggtttg acaggccatc agctgtgaag 2400
accaaagagt caggtgtggg gcagataaat attaacgaca actatctggt tggtctcaaa 2460
atgaggacta tagcttgtgg gccaccacag ttgactgtgg ggctgacagc cagcagaagg 2520
agcgtggggg ttggggatga ccctgtaggg gaatctctgg agaaccccca gcctcaagct 2580
ccacttggaa tgatgactgg cctggatcac tacattgagc gtatccagaa gctgctggca 2640
gaacagcaga cactgctggc tgagaactac agtgaactgg cagaagcttt cggggaacct 2700
cactcacaga tgggctccct caactctcag ctcatcagca ccctgtcgtc tatcaactct 2760
gtcatgaaat ctgcaagcac tgaagagctg aggaaccctg acttccagaa aaccagtctg 2820
ggtaaaatca caggcaatta tttgggatat acctgtaagt gtgggggcct tcagtcagga 2880
agtcccttaa gctcccagac atcccagcct gagcaagaag tggggacctc agaaggaaag 2940
ccaatcagca gcctggatgc cttccccact caggaaggta cgctgtctcc agtgaacctg 3000
acagacgacc agatcgccgc tggcctctat gcatgtacaa acaatgaaag tacactgaag 3060
tccatcatga agaagaaaga tggtaacaaa gattcaaatg gcgcaaaaaa gaatcttcag 3120
tttgttggca ttaatggagg gtatgaaaca acttcaagtg atgattccag ctcagatgaa 3180

agctcttctt ccgagtcaga tgacgagtgt gatgtcattg agtatcctct tgaagaagag 3240
gaggaggagg aggatgaaga cactcgggga atggcagaag ggcaccatgc agttaatatt 3300
gaaggtttga agtctgccag ggtggaagat gaaatgcagg ttcaagaatg tgaacctgag 3360
aaggtggaaa tcagagagag gtatgaatta agtgaaaaga tgttgtctgc atgcaactta 3420
ctgaaaaata ctataaatga ccccaaagct ttgaccagca aagatatgag gttctgtctg 3480
aacaccctcc agcacgagtg gttccgcgtg tccagtcaga agtcagccat tccagccatg 3540
gtgggggact acatagctgc ttttgaggcc atttccccag atgtcctccg ctatgtcatc 3600
aacttggcag acggcaacgg caacacagcc ctccattaca gcgtgtccca ctccaacttc 3660
gagattgtga agctgctgtt agatgccgat gtgtgtaatg tggatcacca gaacaaggca 3720
ggctacaccc ccatcatgtt ggcggccctc gccgctgtgg aagcagagaa ggacatgcgg 3780
attgtggaag aactcttcgg ctgtggggat gtgaatgcca aagctagtca ggcgggacag 3840
acggccctca tgctggcggt cagtcacgga cggatagaca tggtgaaggg ccttctggcc 3900
tgtggggctg atgtcaacat ccaggatgac gagggctcca cggccctcat gtgtgccagc 3960
gagcacgggc acgtggagat tgtcaagctg ctgctggccc agcccggctg caacggtcac 4020
ctagaggaca acgatggcag cactgcgctc tcaatcgccc tggaagcagg acacaaggac 4080
atcgctgttc ttctgtatgc ccatgtcaac tttgcaaaag cccagtctcc gggcacccct 4140
aggcttggaa ggaagacgtc tcctggcccc acccaccgag gttcatttga ttgattgtat 4200
gcaaatagcc ctttatttac atgccactat taagctgcta attgttcctg ttggggtgac 4260
agatactgaa tgtatacgta ttgtgcctga gctcaccagc aaacagaagc atcaagccca 4320
ggggtaaagg ctgaagcttt cacagtgcag agactgctag cctgggcaca cacacctcct 4380
ttctggccgt cttctgtgta gggcacactt taacccagtc tctgttgctg ttgagtctct 4440
gctccgtttt gtacagtcac agggaattct gatctgaagg ggcaccttct gttcactccc 4500
acaaagtggt gtctggttct cactgagacg ttttaagatt tttccacaaa tatttatatg 4560
tactaaatgt ggaaccatta gaaagttctt ccaaaatctc attccagcat agttttggat 4620
ttttcttttg tcttatttta aaataaggaa gtcgagatga ctttgatcat tggtaacttg 4680
ggcctgggcc agacaaagta taaaacttac aaaagaatat tctcatttgg tcttaactag 4740
gtagatgtaa tatatgactt tttataaaaa gggtatctat atgaacttga cacagtattt 4800
tcagcttttg tattccatac taaagccatg aagaactaca cgtaacatca tcatttgtat 4860
taattgcaca actccaatgc taaaggttgg attgtgttag aggaatcggc tctgtatttg 4920
cctctagaga aacacagtgt tctctttgta tttatggatt cctttttacc gtgtcacatt 4980

tactttggtc ctctatgtat ttaaatgttt gaagtgcctt agactcttgc catattttca 5040
aaataaaatt ccattaagct ctaaaaaaaa aaaaaaaaaa aaa 5083
<210> 4
<211> 5263
<212> DNA
<213> Homo sapiens
<400> 4
   agagcctctc tgtagagatg cttaaagtgc tagctttatt taaaaatcag ctggcaaagc 60
   gggagtgaag aacacacatt ttcagaagat accggtttcc tgttttcatt aagaaaacag 120
   ggaatgcgga acagctgagt gcggcggggg tggtgtcact gcagccggag ggagacctcg 180
   ccggtgaaag ctcagcctat ggcatccgag cgtggccggc caagtttaca cgaatgttga 240
   aacttctcct cacggggtga ggatcgaggg cgcccgaggc cgggtccggc tgagctggag 300
   cgagctgtgc ggggcagcgc gggctggcgg ggagcgcggc gggcgccacg tggaaaagca 360
   ggtgatattc tcagtggaga ccaggacaag gaacagaaag acccttactt tgtggagacc 420
   ccctatggtt atcaactaga cttagatttc ctcaaatatg tggatgacat acagaaggga 480
   aataccatca aaagactgaa catccagaag aggcggaagc cgtccgtgcc atgcccagaa 540
   cccaggacca catctggtca gcaaggtata tggacttcca ctgaatccct ctcatcctcc 600
   aacagtgatg acaacaagca gtgccccaac ttcctcatag ccagaagtca agttacatca 660
   actccaatct caaagccacc tccccctctg gagacctcac tcccttttct taccatccca 720
   gaaaatcgac agctgccacc tccctcacca caactcccaa agcataacct tcatgtcacc 780
   aagacactga tggagacccg gagaagactg gaacaggaga gagccaccat gcagatgaca 840
   ccgggtgagt tcagaaggcc caggctggcc agttttggag gcatgggcac cacaagctcc 900
   ctcccttctt ttgtgggttc tggaaaccac aatcctgcca agcaccagct tcagaatgga 960
   taccaaggta atggggatta tggtagctat gccccagctg ctcccaccac ttcctccatg 1020
   gggagctcca tccgccacag ccccctgagc tcagggatct ccaccccagt gaccaacgtg 1080
   agccccatgc acctgcagca catccgcgag cagatggcca ttgctctgaa acgcctgaag 1140
   gagctggagg agcaggtgcg aaccatccct gtgctccagg taaagatctc tgtcttgcaa 1200
   gaagagaaaa ggcagttggt ctcacagctg aaaaaccaaa gggctgcatc ccagatcaat 1260
   gtctgtggtg tgaggaagcg gtcctatagt gcggggaacg cctcccagct ggaacagctc 1320
   tcccgggccc gaagaagtgg cggggaatta tacattgact atgaggagga agaaatggag 1380
   accgtagaac agagcacgca gaggataaag gagttccggc aacttacagc agacatgcaa 1440
   gccctggagc agaagatcca ggacagcagc tgtgaggcct cctcagagct cagggagaat 1500
   ggagagtgcc ggtctgtggc tgtgggtgcc gaggagaaca tgaacgacat cgtcgtgtac 1560
   cacagaggct ccaggtcctg taaggatgca gctgtaggga cacttgttga gatgagaaat 1620
   tgtggggtca gcgtgacaga ggccatgctt ggagtgatga ctgaagctga caaagaaatt 1680
   gagctgcaac agcagaccat agaatccttg aaggaaaaga tctatcgcct agaagtacag 1740
   cttagagaaa ccacccatga ccgggagatg actaaactga aacaagagct gcaggctgct 1800
   ggatcgagga aaaaggttga caaagccacg atggcccagc cgcttgtttt cagtaaggtg 1860
   gtggaggcag tggtgcagac cagagaccaa atggtcggca gtcacatgga cctggtggac 1920
   acgtgtgttg ggacctccgt ggaaacaaac agtgtaggca tctcctgcca gcctgaatgt 1980
   aagaataaag tcgtagggcc tgagctgcct atgaattggt ggattgttaa ggagagggtg 2040
   gaaatgcatg accgatgtgc tgggaggtct gtggaaatgt gtgacaagag tgtgagtgtg 2100
   gaagtcagcg tctgcgaaac aggcagcaac acagaggagt ctgtgaacga cctcacactc 2160
   ctcaagacaa acttgaatct caaagaagtg cggtctatcg gttgtggaga ttgttctgtt 2220
   gacgtgaccg tctgctctcc aaaggagtgc gcctcccggg gcgtgaacac tgaggctgtt 2280
   agccaggtgg aagctgccgt catggcagtg cctcgtactg cagaccagga cactagcaca 2340
   gatttggaac aggtgcacca gttcaccaac accgagacgg ccaccctcat agagtcctgc 2400
   accaacactt gtctaagcac tttggacaag cagaccagca cccagactgt ggagacgcgg 2460
   acagtagctg taggagaagg ccgtgtcaag gacatcaact cctccaccaa gacgcggtcc 2520
   attggtgttg gaacgttgct ttctggccat tctgggtttg acaggccatc agctgtgaag 2580
   accaaagagt caggtgtggg gcagataaat attaacgaca actatctggt tggtctcaaa 2640
   atgaggacta tagcttgtgg gccaccacag ttgactgtgg ggctgacagc cagcagaagg 2700
   agcgtggggg ttggggatga ccctgtaggg gaatctctgg agaaccccca gcctcaagct 2760
   ccacttggaa tgatgactgg cctggatcac tacattgagc gtatccagaa gctgctggca 2820
   gaacagcaga cactgctggc tgagaactac agtgaactgg cagaagcttt cggggaacct 2880
   cactcacaga tgggctccct caactctcag ctcatcagca ccctgtcgtc tatcaactct 2940
   gtcatgaaat ctgcaagcac tgaagagctg aggaaccctg acttccagaa aaccagtctg 3000
   ggtaaaatca caggcaatta tttgggatat acctgtaagt gtgggggcct tcagtcagga 3060
   agtcccttaa gctcccagac atcccagcct gagcaagaag tggggacctc agaaggaaag 3120
   ccaatcagca gcctggatgc cttccccact caggaaggta cgctgtctcc agtgaacctg 3180
   acagacgacc agatcgccgc tggcctctat gcatgtacaa acaatgaaag tacactgaag 3240
   tccatcatga agaagaaaga tggtaacaaa gattcaaatg gcgcaaaaaa gaatcttcag 3300
   tttgttggca ttaatggagg gtatgaaaca acttcaagtg atgattccag ctcagatgaa 3360
   agctcttctt ccgagtcaga tgacgagtgt gatgtcattg agtatcctct tgaagaagag 3420
   gaggaggagg aggatgaaga cactcgggga atggcagaag ggcaccatgc agttaatatt 3480
   gaaggtttga agtctgccag ggtggaagat gaaatgcagg ttcaagaatg tgaacctgag 3540
   aaggtggaaa tcagagagag gtatgaatta agtgaaaaga tgttgtctgc atgcaactta 3600
   ctgaaaaata ctataaatga ccccaaagct ttgaccagca aagatatgag gttctgtctg 3660
   aacaccctcc agcacgagtg gttccgcgtg tccagtcaga agtcagccat tccagccatg 3720
   gtgggggact acatagctgc ttttgaggcc atttccccag atgtcctccg ctatgtcatc 3780
   aacttggcag acggcaacgg caacacagcc ctccattaca gcgtgtccca ctccaacttc 3840
   gagattgtga agctgctgtt agatgccgat gtgtgtaatg tggatcacca gaacaaggca 3900
   ggctacaccc ccatcatgtt ggcggccctc gccgctgtgg aagcagagaa ggacatgcgg 3960
   attgtggaag aactcttcgg ctgtggggat gtgaatgcca aagctagtca ggcgggacag 4020
   acggccctca tgctggcggt cagtcacgga cggatagaca tggtgaaggg ccttctggcc 4080
   tgtggggctg atgtcaacat ccaggatgac gagggctcca cggccctcat gtgtgccagc 4140
   gagcacgggc acgtggagat tgtcaagctg ctgctggccc agcccggctg caacggtcac 4200
   ctagaggaca acgatggcag cactgcgctc tcaatcgccc tggaagcagg acacaaggac 4260
   atcgctgttc ttctgtatgc ccatgtcaac tttgcaaaag cccagtctcc gggcacccct 4320
   aggcttggaa ggaagacgtc tcctggcccc acccaccgag gttcatttga ttgattgtat 4380
   gcaaatagcc ctttatttac atgccactat taagctgcta attgttcctg ttggggtgac 4440
   agatactgaa tgtatacgta ttgtgcctga gctcaccagc aaacagaagc atcaagccca 4500
   ggggtaaagg ctgaagcttt cacagtgcag agactgctag cctgggcaca cacacctcct 4560
   ttctggccgt cttctgtgta gggcacactt taacccagtc tctgttgctg ttgagtctct 4620
   gctccgtttt gtacagtcac agggaattct gatctgaagg ggcaccttct gttcactccc 4680
   acaaagtggt gtctggttct cactgagacg ttttaagatt tttccacaaa tatttatatg 4740
   tactaaatgt ggaaccatta gaaagttctt ccaaaatctc attccagcat agttttggat 4800
   ttttcttttg tcttatttta aaataaggaa gtcgagatga ctttgatcat tggtaacttg 4860
   ggcctgggcc agacaaagta taaaacttac aaaagaatat tctcatttgg tcttaactag 4920
   gtagatgtaa tatatgactt tttataaaaa gggtatctat atgaacttga cacagtattt 4980
   tcagcttttg tattccatac taaagccatg aagaactaca cgtaacatca tcatttgtat 5040
   taattgcaca actccaatgc taaaggttgg attgtgttag aggaatcggc tctgtatttg 5100
   cctctagaga aacacagtgt tctctttgta tttatggatt cctttttacc gtgtcacatt 5160
   tactttggtc ctctatgtat ttaaatgttt gaagtgcctt agactcttgc catattttca 5220
   aaataaaatt ccattaagct ctaaaaaaaa aaaaaaaaaa aaa 5263
<210> 5
<211> 1352
<212> PRT
<213> Homo sapiens
<400> 5
<210> 6
<211> 1194
<212> PRT
<213> Homo sapiens
<400> 6
   Met Glu Thr Arg Arg Arg Leu Glu Gln Glu Arg Ala Thr Met Gln Met
   1 5 10 15
   Thr Pro Gly Glu Phe Arg Arg Pro Arg Leu Ala Ser Phe Gly Gly Met
   20 25 30
   Gly Thr Thr Ser Ser Leu Pro Ser Phe Val Gly Ser Gly Asn His Asn
   35 40 45
   Pro Ala Lys His Gln Leu Gln Asn Gly Tyr Gln Gly Asn Gly Asp Tyr
   50 55 60
   Gly Ser Tyr Ala Pro Ala Ala Pro Thr Thr Ser Ser Met Gly Ser Ser
   65 70 75 80
   Ile Arg His Ser Pro Leu Ser Ser Gly Ile Ser Thr Pro Val Thr Asn
   85 90 95
   Val Ser Pro Met His Leu Gln His Ile Arg Glu Gln Met Ala Ile Ala
   100 105 110
   Leu Lys Arg Leu Lys Glu Leu Glu Glu Gln Val Arg Thr Ile Pro Val
   115 120 125
   Leu Gln Val Lys Ile Ser Val Leu Gln Glu Glu Lys Arg Gln Leu Val
   130 135 140
   Ser Gln Leu Lys Asn Gln Arg Ala Ala Ser Gln Ile Asn Val Cys Gly
   145 150 155 160
   Val Arg Lys Arg Ser Tyr Ser Ala Gly Asn Ala Ser Gln Leu Glu Gln
   165 170 175
   Leu Ser Arg Ala Arg Arg Ser Gly Gly Glu Leu Tyr Ile Asp Tyr Glu
   180 185 190
   Glu Glu Glu Met Glu Thr Val Glu Gln Ser Thr Gln Arg Ile Lys Glu
   195 200 205
   Phe Arg Gln Leu Thr Ala Asp Met Gln Ala Leu Glu Gln Lys Ile Gln
   210 215 220
   Asp Ser Ser Cys Glu Ala Ser Ser Glu Leu Arg Glu Asn Gly Glu Cys
   225 230 235 240
   Arg Ser Val Ala Val Gly Ala Glu Glu Asn Met Asn Asp Ile Val Val
   245 250 255
   Tyr His Arg Gly Ser Arg Ser Cys Lys Asp Ala Ala Val Gly Thr Leu
   260 265 270
   Val Glu Met Arg Asn Cys Gly Val Ser Val Thr Glu Ala Met Leu Gly
   275 280 285
   Val Met Thr Glu Ala Asp Lys Glu Ile Glu Leu Gln Gln Gln Thr Ile
   290 295 300
   Glu Ser Leu Lys Glu Lys Ile Tyr Arg Leu Glu Val Gln Leu Arg Glu
   305 310 315 320
   Thr Thr His Asp Arg Glu Met Thr Lys Leu Lys Gln Glu Leu Gln Ala
   325 330 335
   Ala Gly Ser Arg Lys Lys Val Asp Lys Ala Thr Met Ala Gln Pro Leu
   340 345 350
   Val Phe Ser Lys Val Val Glu Ala Val Val Gln Thr Arg Asp Gln Met
   355 360 365
   Val Gly Ser His Met Asp Leu Val Asp Thr Cys Val Gly Thr Ser Val
   370 375 380
   Glu Thr Asn Ser Val Gly Ile Ser Cys Gln Pro Glu Cys Lys Asn Lys
   385 390 395 400
   Val Val Gly Pro Glu Leu Pro Met Asn Trp Trp Ile Val Lys Glu Arg
   405 410 415
   Val Glu Met His Asp Arg Cys Ala Gly Arg Ser Val Glu Met Cys Asp
   420 425 430
   Lys Ser Val Ser Val Glu Val Ser Val Cys Glu Thr Gly Ser Asn Thr
   435 440 445
   Glu Glu Ser Val Asn Asp Leu Thr Leu Leu Lys Thr Asn Leu Asn Leu
   450 455 460
   Lys Glu Val Arg Ser Ile Gly Cys Gly Asp Cys Ser Val Asp Val Thr
   465 470 475 480
   Val Cys Ser Pro Lys Glu Cys Ala Ser Arg Gly Val Asn Thr Glu Ala
   485 490 495
   Val Ser Gln Val Glu Ala Ala Val Met Ala Val Pro Arg Thr Ala Asp
   500 505 510
   Gln Asp Thr Ser Thr Asp Leu Glu Gln Val His Gln Phe Thr Asn Thr
   515 520 525
   Glu Thr Ala Thr Leu Ile Glu Ser Cys Thr Asn Thr Cys Leu Ser Thr
   530 535 540
   Leu Asp Lys Gln Thr Ser Thr Gln Thr Val Glu Thr Arg Thr Val Ala
   545 550 555 560
   Val Gly Glu Gly Arg Val Lys Asp Ile Asn Ser Ser Thr Lys Thr Arg
   565 570 575
   Ser Ile Gly Val Gly Thr Leu Leu Ser Gly His Ser Gly Phe Asp Arg
   580 585 590
   Pro Ser Ala Val Lys Thr Lys Glu Ser Gly Val Gly Gln Ile Asn Ile
   595 600 605
   Asn Asp Asn Tyr Leu Val Gly Leu Lys Met Arg Thr Ile Ala Cys Gly
   610 615 620
   Pro Pro Gln Leu Thr Val Gly Leu Thr Ala Ser Arg Arg Ser Val Gly
   625 630 635 640
   Val Gly Asp Asp Pro Val Gly Glu Ser Leu Glu Asn Pro Gln Pro Gln
   645 650 655
   Ala Pro Leu Gly Met Met Thr Gly Leu Asp His Tyr Ile Glu Arg Ile
   660 665 670
   Gln Lys Leu Leu Ala Glu Gln Gln Thr Leu Leu Ala Glu Asn Tyr Ser
   675 680 685
   Glu Leu Ala Glu Ala Phe Gly Glu Pro His Ser Gln Met Gly Ser Leu
   690 695 700
   Asn Ser Gln Leu Ile Ser Thr Leu Ser Ser Ile Asn Ser Val Met Lys
   705 710 715 720
   Ser Ala Ser Thr Glu Glu Leu Arg Asn Pro Asp Phe Gln Lys Thr Ser
   725 730 735
   Leu Gly Lys Ile Thr Gly Asn Tyr Leu Gly Tyr Thr Cys Lys Cys Gly
   740 745 750
   Gly Leu Gln Ser Gly Ser Pro Leu Ser Ser Gln Thr Ser Gln Pro Glu
   755 760 765
   Gln Glu Val Gly Thr Ser Glu Gly Lys Pro Ile Ser Ser Leu Asp Ala
   770 775 780
   Phe Pro Thr Gln Glu Gly Thr Leu Ser Pro Val Asn Leu Thr Asp Asp
   785 790 795 800
   Gln Ile Ala Ala Gly Leu Tyr Ala Cys Thr Asn Asn Glu Ser Thr Leu
   805 810 815
   Lys Ser Ile Met Lys Lys Lys Asp Gly Asn Lys Asp Ser Asn Gly Ala
   820 825 830
   Lys Lys Asn Leu Gln Phe Val Gly Ile Asn Gly Gly Tyr Glu Thr Thr
   835 840 845
   Ser Ser Asp Asp Ser Ser Ser Asp Glu Ser Ser Ser Ser Glu Ser Asp
   850 855 860
   Asp Glu Cys Asp Val Ile Glu Tyr Pro Leu Glu Glu Glu Glu Glu Glu
   865 870 875 880
   Glu Asp Glu Asp Thr Arg Gly Met Ala Glu Gly His His Ala Val Asn
   885 890 895
   Ile Glu Gly Leu Lys Ser Ala Arg Val Glu Asp Glu Met Gln Val Gln
   900 905 910
   Glu Cys Glu Pro Glu Lys Val Glu Ile Arg Glu Arg Tyr Glu Leu Ser
   915 920 925
   Glu Lys Met Leu Ser Ala Cys Asn Leu Leu Lys Asn Thr Ile Asn Asp
   930 935 940
   Pro Lys Ala Leu Thr Ser Lys Asp Met Arg Phe Cys Leu Asn Thr Leu
   945 950 955 960
   Gln His Glu Trp Phe Arg Val Ser Ser Gln Lys Ser Ala Ile Pro Ala
   965 970 975
   Met Val Gly Asp Tyr Ile Ala Ala Phe Glu Ala Ile Ser Pro Asp Val
   980 985 990
   Leu Arg Tyr Val Ile Asn Leu Ala Asp Gly Asn Gly Asn Thr Ala Leu
   995 1000 1005
   His Tyr Ser Val Ser His Ser Asn Phe Glu Ile Val Lys Leu Leu
   1010 1015 1020
   Leu Asp Ala Asp Val Cys Asn Val Asp His Gln Asn Lys Ala Gly
   1025 1030 1035
   Tyr Thr Pro Ile Met Leu Ala Ala Leu Ala Ala Val Glu Ala Glu
   1040 1045 1050
   Lys Asp Met Arg Ile Val Glu Glu Leu Phe Gly Cys Gly Asp Val
   1055 1060 1065
   Asn Ala Lys Ala Ser Gln Ala Gly Gln Thr Ala Leu Met Leu Ala
   1070 1075 1080
   Val Ser His Gly Arg Ile Asp Met Val Lys Gly Leu Leu Ala Cys
   1085 1090 1095
   Gly Ala Asp Val Asn Ile Gln Asp Asp Glu Gly Ser Thr Ala Leu
   1100 1105 1110
   Met Cys Ala Ser Glu His Gly His Val Glu Ile Val Lys Leu Leu
   1115 1120 1125
   Leu Ala Gln Pro Gly Cys Asn Gly His Leu Glu Asp Asn Asp Gly
   1130                 1135                 1140
   Ser Thr Ala Leu Ser Ile Ala Leu Glu Ala Gly His Lys Asp Ile
   1145                 1150                 1155
   Ala Val Leu Leu Tyr Ala His Val Asn Phe Ala Lys Ala Gln Ser
   1160                 1165                 1170
   Pro Gly Thr Pro Arg Leu Gly Arg Lys Thr Ser Pro Gly Pro Thr
   1175                 1180                 1185
   His Arg Gly Ser Phe Asp
   1190

<210> 7

<211> 4138

<212> DNA

<213> Homo sapiens

<400> 7

cttctgtgct gttccttctt gcctctaact tgtaaacaag acgtactagg acgatgctaa 60

tggaaagtca caaaccgctg ggtttttgaa aggatccttg ggacctcatg cacatttgtg 120

gaaactggat ggagagattt ggggaagcat ggactcttta gccagcttag ttctctgtgg 180

agtcagcttg ctcctttctg gaactgtgga aggtgccatg gacttgatct tgatcaattc 240

cctacctctt gtatctgatg ctgaaacatc tctcacctgc attgcctctg ggtggcgccc 300

ccatgagccc atcaccatag gaagggactt tgaagcctta atgaaccagc accaggatcc 360

gctggaagtt actcaagatg tgaccagaga atgggctaaa aaagttgttt ggaagagaga 420

aaaggctagt aagatcaatg gtgcttattt ctgtgaaggg cgagttcgag gagaggcaat 480

caggatacga accatgaaga tgcgtcaaca agcttccttc ctaccagcta ctttaactat 540

gactgtggac aagggagata acgtgaacat atctttcaaa aaggtattga ttaaagaaga 600

agatgcagtg atttacaaaa atggttcctt catccattca gtgccccggc atgaagtacc 660

tgatattcta gaagtacacc tgcctcatgc tcagccccag gatgctggag tgtactcggc 720

caggtatata ggaggaaacc tcttcacctc ggccttcacc aggctgatag tccggagatg 780

tgaagcccag aagtggggac ctgaatgcaa ccatctctgt actgcttgta tgaacaatgg 840

tgtctgccat gaagatactg gagaatgcat ttgccctcct gggtttatgg gaaggacgtg 900

tgagaaggct tgtgaactgc acacgtttgg cagaacttgt aaagaaaggt gcagtggaca 960

agagggatgc aagtcttatg tgttctgtct ccctgacccc tatgggtgtt cctgtgccac 1020
aggctggaag ggtctgcagt gcaatgaagc atgccaccct ggtttttacg ggccagattg 1080
taagcttagg tgcagctgca acaatgggga gatgtgtgat cgcttccaag gatgtctctg 1140
ctctccagga tggcaggggc tccagtgtga gagagaaggc ataccgagga tgaccccaaa 1200
gatagtggat ttgccagatc atatagaagt aaacagtggt aaatttaatc ccatttgcaa 1260
agcttctggc tggccgctac ctactaatga agaaatgacc ctggtgaagc cggatgggac 1320
agtgctccat ccaaaagact ttaaccatac ggatcatttc tcagtagcca tattcaccat 1380
ccaccggatc ctcccccctg actcaggagt ttgggtctgc agtgtgaaca cagtggctgg 1440
gatggtggaa aagcccttca acatttctgt taaagttctt ccaaagcccc tgaatgcccc 1500
aaacgtgatt gacactggac ataactttgc tgtcatcaac atcagctctg agccttactt 1560
tggggatgga ccaatcaaat ccaagaagct tctatacaaa cccgttaatc actatgaggc 1620
ttggcaacat attcaagtga caaatgagat tgttacactc aactatttgg aacctcggac 1680
agaatatgaa ctctgtgtgc aactggtccg tcgtggagag ggtggggaag ggcatcctgg 1740
acctgtgaga cgcttcacaa cagcttctat cggactccct cctccaagag gtctaaatct 1800
cctgcctaaa agtcagacca ctctaaattt gacctggcaa ccaatatttc caagctcgga 1860
agatgacttt tatgttgaag tggagagaag gtctgtgcaa aaaagtgatc agcagaatat 1920
taaagttcca ggcaacttga cttcggtgct acttaacaac ttacatccca gggagcagta 1980
cgtggtccga gctagagtca acaccaaggc ccagggggaa tggagtgaag atctcactgc 2040
ttggaccctt agtgacattc ttcctcctca accagaaaac atcaagattt ccaacattac 2100
acactcctcg gctgtgattt cttggacaat attggatggc tattctattt cttctattac 2160
tatccgttac aaggttcaag gcaagaatga agaccagcac gttgatgtga agataaagaa 2220
tgccaccatc attcagtatc agctcaaggg cctagagcct gaaacagcat accaggtgga 2280
catttttgca gagaacaaca tagggtcaag caacccagcc ttttctcatg aactggtgac 2340
cctcccagaa tctcaagcac cagcggacct cggagggggg aagatgctgc ttatagccat 2400
ccttggctct gctggaatga cctgcctgac tgtgctgttg gcctttctga tcatattgca 2460
attgaagagg gcaaatgtgc aaaggagaat ggcccaagcc ttccaaaacg tgagggaaga 2520
accagctgtg cagttcaact cagggactct ggccctaaac aggaaggtca aaaacaaccc 2580
agatcctaca atttatccag tgcttgactg gaatgacatc aaatttcaag atgtgattgg 2640
ggagggcaat tttggccaag ttcttaaggc gcgcatcaag aaggatgggt tacggatgga 2700
tgctgccatc aaaagaatga aagaatatgc ctccaaagat gatcacaggg actttgcagg 2760

agaactggaa gttctttgta aacttggaca ccatccaaac atcatcaatc tcttaggagc 2820
atgtgaacat cgaggctact tgtacctggc cattgagtac gcgccccatg gaaaccttct 2880
ggacttcctt cgcaagagcc gtgtgctgga gacggaccca gcatttgcca ttgccaatag 2940
caccgcgtcc acactgtcct cccagcagct ccttcacttc gctgccgacg tggcccgggg 3000
catggactac ttgagccaaa aacagtttat ccacagggat ctggctgcca gaaacatttt 3060
agttggtgaa aactatgtgg caaaaatagc agattttgga ttgtcccgag gtcaagaggt 3120
gtacgtgaaa aagacaatgg gaaggctccc agtgcgctgg atggccatcg agtcactgaa 3180
ttacagtgtg tacacaacca acagtgatgt atggtcctat ggtgtgttac tatgggagat 3240
tgttagctta ggaggcacac cctactgcgg gatgacttgt gcagaactct acgagaagct 3300
gccccagggc tacagactgg agaagcccct gaactgtgat gatgaggtgt atgatctaat 3360
gagacaatgc tggcgggaga agccttatga gaggccatca tttgcccaga tattggtgtc 3420
cttaaacaga atgttagagg agcgaaagac ctacgtgaat accacgcttt atgagaagtt 3480
tacttatgca ggaattgact gttctgctga agaagcggcc taggacagaa catctgtata 3540
ccctctgttt ccctttcact ggcatgggag acccttgaca actgctgaga aaacatgcct 3600
ctgccaaagg atgtgatata taagtgtaca tatgtgctgg aattctaaca agtcataggt 3660
taatatttaa gacactgaaa aatctaagtg atataaatca gattcttctc tctcatttta 3720
tccctcacct gtagcatgcc agtcccgttt catttagtca tgtgaccact ctgtcttgtg 3780
tttccacagc ctgcaagttc agtccaggat gctaacatct aaaaatagac ttaaatctca 3840
ttgcttacaa gcctaagaat ctttagagaa gtatacataa gtttaggata aaataatggg 3900
attttctttt cttttctctg gtaatattga cttgtatatt ttaagaaata acagaaagcc 3960
tgggtgacat ttgggagaca tgtgacattt atatattgaa ttaatatccc tacatgtatt 4020
gcacattgta aaaagtttta gttttgatga gttgtgagtt taccttgtat actgtaggca 4080
cactttgcac tgatatatca tgagtgaata aatgtcttgc ctactcaaaa aaaaaaaa 4138
<210> 8
<211> 1124
<212> PRT
<213> Homo sapiens
<400> 8
Met Asp Ser Leu Ala Ser Leu Val Leu Cys Gly Val Ser Leu Leu Leu
1               5                   10                  15

Ser Gly Thr Val Glu Gly Ala Met Asp Leu Ile Leu Ile Asn Ser Leu
            20                  25                  30
Pro Leu Val Ser Asp Ala Glu Thr Ser Leu Thr Cys Ile Ala Ser Gly
        35                  40                  45
Trp Arg Pro His Glu Pro Ile Thr Ile Gly Arg Asp Phe Glu Ala Leu
    50                  55                  60
Met Asn Gln His Gln Asp Pro Leu Glu Val Thr Gln Asp Val Thr Arg
65                  70                  75                  80
Glu Trp Ala Lys Lys Val Val Trp Lys Arg Glu Lys Ala Ser Lys Ile
                85                  90                  95
Asn Gly Ala Tyr Phe Cys Glu Gly Arg Val Arg Gly Glu Ala Ile Arg
            100                 105                 110
Ile Arg Thr Met Lys Met Arg Gln Gln Ala Ser Phe Leu Pro Ala Thr
        115                 120                 125
Leu Thr Met Thr Val Asp Lys Gly Asp Asn Val Asn Ile Ser Phe Lys
    130                 135                 140
Lys Val Leu Ile Lys Glu Glu Asp Ala Val Ile Tyr Lys Asn Gly Ser
145                 150                 155                 160
Phe Ile His Ser Val Pro Arg His Glu Val Pro Asp Ile Leu Glu Val
                165                 170                 175
His Leu Pro His Ala Gln Pro Gln Asp Ala Gly Val Tyr Ser Ala Arg
            180                 185                 190
Tyr Ile Gly Gly Asn Leu Phe Thr Ser Ala Phe Thr Arg Leu Ile Val
        195                 200                 205
Arg Arg Cys Glu Ala Gln Lys Trp Gly Pro Glu Cys Asn His Leu Cys
    210                 215                 220
Thr Ala Cys Met Asn Asn Gly Val Cys His Glu Asp Thr Gly Glu Cys
225                 230                 235                 240
Ile Cys Pro Pro Gly Phe Met Gly Arg Thr Cys Glu Lys Ala Cys Glu
                245                 250                 255

Leu His Thr Phe Gly Arg Thr Cys Lys Glu Arg Cys Ser Gly Gln Glu
            260                 265                 270
Gly Cys Lys Ser Tyr Val Phe Cys Leu Pro Asp Pro Tyr Gly Cys Ser
        275                 280                 285
Cys Ala Thr Gly Trp Lys Gly Leu Gln Cys Asn Glu Ala Cys His Pro
    290                 295                 300
Gly Phe Tyr Gly Pro Asp Cys Lys Leu Arg Cys Ser Cys Asn Asn Gly
305                 310                 315                 320
Glu Met Cys Asp Arg Phe Gln Gly Cys Leu Cys Ser Pro Gly Trp Gln
                325                 330                 335
Gly Leu Gln Cys Glu Arg Glu Gly Ile Pro Arg Met Thr Pro Lys Ile
            340                 345                 350
Val Asp Leu Pro Asp His Ile Glu Val Asn Ser Gly Lys Phe Asn Pro
        355                 360                 365
Ile Cys Lys Ala Ser Gly Trp Pro Leu Pro Thr Asn Glu Glu Met Thr
    370                 375                 380
Leu Val Lys Pro Asp Gly Thr Val Leu His Pro Lys Asp Phe Asn His
385                 390                 395                 400
Thr Asp His Phe Ser Val Ala Ile Phe Thr Ile His Arg Ile Leu Pro
                405                 410                 415
Pro Asp Ser Gly Val Trp Val Cys Ser Val Asn Thr Val Ala Gly Met
            420                 425                 430
Val Glu Lys Pro Phe Asn Ile Ser Val Lys Val Leu Pro Lys Pro Leu
        435                 440                 445
Asn Ala Pro Asn Val Ile Asp Thr Gly His Asn Phe Ala Val Ile Asn
    450                 455                 460
Ile Ser Ser Glu Pro Tyr Phe Gly Asp Gly Pro Ile Lys Ser Lys Lys
465                 470                 475                 480
Leu Leu Tyr Lys Pro Val Asn His Tyr Glu Ala Trp Gln His Ile Gln
                485                 490                 495

Val Thr Asn Glu Ile Val Thr Leu Asn Tyr Leu Glu Pro Arg Thr Glu
            500                 505                 510
Tyr Glu Leu Cys Val Gln Leu Val Arg Arg Gly Glu Gly Gly Glu Gly
        515                 520                 525
His Pro Gly Pro Val Arg Arg Phe Thr Thr Ala Ser Ile Gly Leu Pro
    530                 535                 540
Pro Pro Arg Gly Leu Asn Leu Leu Pro Lys Ser Gln Thr Thr Leu Asn
545                 550                 555                 560
Leu Thr Trp Gln Pro Ile Phe Pro Ser Ser Glu Asp Asp Phe Tyr Val
                565                 570                 575
Glu Val Glu Arg Arg Ser Val Gln Lys Ser Asp Gln Gln Asn Ile Lys
            580                 585                 590
Val Pro Gly Asn Leu Thr Ser Val Leu Leu Asn Asn Leu His Pro Arg
        595                 600                 605
Glu Gln Tyr Val Val Arg Ala Arg Val Asn Thr Lys Ala Gln Gly Glu
    610                 615                 620
Trp Ser Glu Asp Leu Thr Ala Trp Thr Leu Ser Asp Ile Leu Pro Pro
625                 630                 635                 640
Gln Pro Glu Asn Ile Lys Ile Ser Asn Ile Thr His Ser Ser Ala Val
                645                 650                 655
Ile Ser Trp Thr Ile Leu Asp Gly Tyr Ser Ile Ser Ser Ile Thr Ile
            660                 665                 670
Arg Tyr Lys Val Gln Gly Lys Asn Glu Asp Gln His Val Asp Val Lys
        675                 680                 685
Ile Lys Asn Ala Thr Ile Ile Gln Tyr Gln Leu Lys Gly Leu Glu Pro
    690                 695                 700
Glu Thr Ala Tyr Gln Val Asp Ile Phe Ala Glu Asn Asn Ile Gly Ser
705                 710                 715                 720
Ser Asn Pro Ala Phe Ser His Glu Leu Val Thr Leu Pro Glu Ser Gln
                725                 730                 735

Ala Pro Ala Asp Leu Gly Gly Gly Lys Met Leu Leu Ile Ala Ile Leu
            740                 745                 750
Gly Ser Ala Gly Met Thr Cys Leu Thr Val Leu Leu Ala Phe Leu Ile
        755                 760                 765
Ile Leu Gln Leu Lys Arg Ala Asn Val Gln Arg Arg Met Ala Gln Ala
    770                 775                 780
Phe Gln Asn Val Arg Glu Glu Pro Ala Val Gln Phe Asn Ser Gly Thr
785                 790                 795                 800
Leu Ala Leu Asn Arg Lys Val Lys Asn Asn Pro Asp Pro Thr Ile Tyr
                805                 810                 815
Pro Val Leu Asp Trp Asn Asp Ile Lys Phe Gln Asp Val Ile Gly Glu
            820                 825                 830
Gly Asn Phe Gly Gln Val Leu Lys Ala Arg Ile Lys Lys Asp Gly Leu
        835                 840                 845
Arg Met Asp Ala Ala Ile Lys Arg Met Lys Glu Tyr Ala Ser Lys Asp
    850                 855                 860
Asp His Arg Asp Phe Ala Gly Glu Leu Glu Val Leu Cys Lys Leu Gly
865                 870                 875                 880
His His Pro Asn Ile Ile Asn Leu Leu Gly Ala Cys Glu His Arg Gly
                885                 890                 895
Tyr Leu Tyr Leu Ala Ile Glu Tyr Ala Pro His Gly Asn Leu Leu Asp
            900                 905                 910
Phe Leu Arg Lys Ser Arg Val Leu Glu Thr Asp Pro Ala Phe Ala Ile
        915                 920                 925
Ala Asn Ser Thr Ala Ser Thr Leu Ser Ser Gln Gln Leu Leu His Phe
    930                 935                 940
Ala Ala Asp Val Ala Arg Gly Met Asp Tyr Leu Ser Gln Lys Gln Phe
945                 950                 955                 960
Ile His Arg Asp Leu Ala Ala Arg Asn Ile Leu Val Gly Glu Asn Tyr
                965                 970                 975

Val Ala Lys Ile Ala Asp Phe Gly Leu Ser Arg Gly Gln Glu Val Tyr
            980                 985                 990
Val Lys Lys Thr Met Gly Arg Leu Pro Val Arg Trp Met Ala Ile Glu
        995                 1000                1005
Ser Leu Asn Tyr Ser Val Tyr Thr Thr Asn Ser Asp Val Trp Ser
    1010                1015                1020
Tyr Gly Val Leu Leu Trp Glu Ile Val Ser Leu Gly Gly Thr Pro
    1025                1030                1035
Tyr Cys Gly Met Thr Cys Ala Glu Leu Tyr Glu Lys Leu Pro Gln
    1040                1045                1050
Gly Tyr Arg Leu Glu Lys Pro Leu Asn Cys Asp Asp Glu Val Tyr
    1055                1060                1065
Asp Leu Met Arg Gln Cys Trp Arg Glu Lys Pro Tyr Glu Arg Pro
    1070                1075                1080
Ser Phe Ala Gln Ile Leu Val Ser Leu Asn Arg Met Leu Glu Glu
    1085                1090                1095
Arg Lys Thr Tyr Val Asn Thr Thr Leu Tyr Glu Lys Phe Thr Tyr
    1100                1105                1110
Ala Gly Ile Asp Cys Ser Ala Glu Glu Ala Ala
    1115                1120
<210> 9
<211> 1746
<212> DNA
<213> Homo sapiens
<400> 9
tgcacagcct ctcttaaaac aacatgccac agttaaaact tacttcagtt tggacaacta 60
ctcacagcta ctacacagag acccgaacga gtcactgata tacacctgga ccaccaccaa 120
tggatataca aatggcaaac aattttactc cgccctctgc aactcctcag ggaaatgact 180
gtgacctcta tgcacatcac agcacggcca ggatagtaat gcctctgcat tacagcctcg 240
tcttcatcat tgggctcgtg ggaaacttac tagccttggt cgtcattgtt caaaacagga 300

aaaaaatcaa ctctaccacc ctctattcaa caaatttggt gatttctgat atacttttta 360
ccaccgcttt gcctacacga atagcctact atgcaatggg ctttgactgg agaatcggag 420
atgccttgtg taggataact gcgctagtgt tttacatcaa cacatatgca ggtgtgaact 480
ttatgacctg cctgagtatt gaccgcttca ttgctgtggt gcaccctcta cgctacaaca 540
agataaaaag gattgaacat gcaaaaggcg tgtgcatatt tgtctggatt ctagtatttg 600
ctcagacact cccactcctc atcaacccta tgtcaaagca ggaggctgaa aggattacat 660
gcatggagta tccaaacttt gaagaaacta aatctcttcc ctggattctg cttggggcat 720
gtttcatagg atatgtactt ccacttataa tcattctcat ctgctattct cagatctgct 780
gcaaactctt cagaactgcc aaacaaaacc cactcactga gaaatctggt gtaaacaaaa 840
aggctctcaa cacaattatt cttattattg ttgtgtttgt tctctgtttc acaccttacc 900
atgttgcaat tattcaacat atgattaaga agcttcgttt ctctaatttc ctggaatgta 960
gccaaagaca ttcgttccag atttctctgc actttacagt atgcctgatg aacttcaatt 1020
gctgcatgga cccttttatc tacttctttg catgtaaagg gtataagaga aaggttatga 1080
ggatgctgaa acggcaagtc agtgtatcga tttctagtgc tgtgaagtca gcccctgaag 1140
aaaattcacg tgaaatgaca gaaacgcaga tgatgataca ttccaagtct tcaaatggaa 1200
agtgaaatgg attgtatttt ggtttatagt gacgtaaact gtatgacaaa ctttgcagga 1260
cttcccttat aaagcaaaat aattgttcag cttccaatta gtattctttt atatttcttt 1320
cattgggcac tttcccatct ccaactcgga agtaagccca agagaacaac ataaagcaaa 1380
caacataaag cacaataaaa atgcaaataa atatttcatt tttatttgta aacgaataca 1440
ccaaaaggag gcgctcttaa taactcccaa tgtaaaaagt tttgttttaa taaaaaattt 1500
aattattatt tcttgccaac aaatggctag aaaggactga atagattata tattgccaga 1560
tgttaatact gtaacatact ttttaaataa catatttctt aaatccaaat ttctctcaat 1620
gttagattta attccctcaa taacaccaat gttttgtttt gtttcgttct gggtcataaa 1680
actttgttaag gaactcttt tggaataaag agcaggatgc tgcaaagtta aaaaaaaaaa 1740
aaaaaa 1746
<210> 10
<211> 361
<212> PRT
<213> Homo sapiens
<400> 10

## Description

### Technical Field

The present invention relates to a method for diagnosing cancer by analyzing or detecting a cancer-associated gene or a product thereof in a specimen.

### Background Art

It has been known that onset of cancer is induced by mutation or quantitative change of a cell protein. Along with recent development in genetic engineering, it has become possible to amplify a gene encoding a specific protein and to analyze gene mutation in cancer cells, resulting in breakthroughs in the field of cancer research. Hitherto, analysis and identification of oncogenes involved in the oncogenic transformation of cells and the abnormal growth of cancer cells have made progress. Meanwhile, in recent years, cancer-suppressing genes have been gaining attention. Mutation or the decreased expression level of cancer-suppressing gene leads to oncogenic transformation of cells. Examples of cancer-suppressing genes that have been identified include Rb gene of retinoblastoma, p53 gene and APC gene of large-bowel cancer, and WT1 gene of Wilms tumor, For instance, an example of a cancer-suppressing agent that uses WT1 gene has been reported (WO2003/002142),

In addition, it has been gradually revealed that cancer development, malignant progression, and metastasis are caused by abnormalities of not only a single gene but also a plurality of genes. In addition, a greater number of unidentified oncogenes and cancer-suppressing genes are now believed to exist. There are many genes known to have effects that suppress cancer. In most cases, screening for such genes has been carried out by an approach of visually detecting mutation of a patient's gene via staining of chromosomal DNA (Yasuhide Yamashita, et al., World J Gastroenterol, 11 (33): 5129-5135, 2005) or by a method wherein a region of gene deletion is roughly selected based on LOH (loss of heterozygosity) analysis so that important gene regions are narrowed down (WO01/032859). However, such methods are not sufficient as means of discovering cancer-suppressing genes. This is because a tremendous number of DNA deletion regions are detected, so that narrowing them down into important gene regions is extremely time- and labor-consuming, which has been a drawback. Further, conventional separation and discrimination methods for pathological conditions of cancer have only been able to determine malignancy with difficulty.

### Disclosure of the Invention

It is an object of the present invention to provide a novel method for diagnosing cancer by discovering a cancer-associated gene which could not be detected by a conventional technique, and detecting deletion, mutation, or an abnormal expression level of such cancer-associated gene.

To achieve the above objects, the present inventors have intensively searched for partially deleted DNA regions in non-small cell lung cancer cases and identified genes differing in methylation degree. Non-small cell lung cancer is further classified by tissue type, including adenocarcinoma, planocellular carcinoma, large cell carcinoma, squamous cell carcinoma, and the like, and it accounts for 80% or more of lung cancer cases. In order to specify deleted DNA in non-small cell lung cancer, the present inventors have screened for genes deleted at high frequencies in cancer via a newly developed array CGH method (Inazawa J., et al., Cancer Sci. 95 (7), 559, 2004). As a result, the present inventors succeeded in isolating a gene that is homozygously deleted in genomic DNA, i.e., a gene that lacks genomic region in the non-small cell lung cancer cell line. Detection of the existence of such gene enabled cancer diagnosis. The present invention has been completed based on such finding.

The present invention provides a method for diagnosing cancer which comprises a step of detecting deletion of the GMDS, ANKRD15, TEK, or EBI2 gene in a test sample by using DNA containing all or part of said gene.

The present invention further provides a method for diagnosing cancer which comprises a step of analyzing the GMDS, ANKRD15, TEK, or EBI2 gene in a test sample by using DNA or RNA containing all or part of the GMDS, ANKRD15, TEK, or EBI2 gene.

Preferably, the analysis involves detection of mutation of the gene or detection of abnormal expression level of the gene.

The present invention provides a method for diagnosing cancer which comprises a step of analyzing GMDS, ANKRD15, TEK, or EBI2 protein in a test sample using an antibody against GMDS, ANKRD15, TEK, OR EBI2 protein or fragment thereof.

Preferably, the analysis involves detection of abnormal expression level of the protein.

Preferably in the method of the present invention, lung cancer is diagnosed.

### Best Mode for Carrying Out the Invention

Hereafter, the embodiments and implementation of the present invention will be described in detail.

The cancer-associated gene, which is targeted in the method for diagnosing cancer according to the present invention, is the GMDS gene, the ANKRD 15 gene, the TEK gene, or the EBI2 gene.

The nucleotide sequences of the GMDS gene, the ANKRD15 gene, the TEK gene, and the EBI2 gene and the amino acid sequences of the GMDS protein, the ANKRD15 protein, the TEK protein, and the EBI2 protein are already known. Such sequence information is available from the database of the National Center for Biotechnology Information (NCBI).

### GMDS:

GMDS is known as GMD or GDP-mannose 4,6-dehydratase (EC 4.2.1.47) (GDP-D-mannose dehydratase), and it has the RefSeq ID of NM_001500 (SEQ ID NO: 1). The amino acid sequence thereof is shown (SEQ ID NO: 2). In the human genome, it is contained in clone RP11-79M24 (available from the UCSC genome browser (http://genome.ucsc.edu/cgi-bin/hgGateway)).

### ANKRD15:

ANKRD15 is known as DKFZp451G231, KANK, KIAA0172, MGC43128, or the ankyrin repeat domain 15, and it has RefSeq IDs of NM_015158 and NM_153186 (SEQ ID NO: 3 and SEQ ID NO: 4). The amino acid sequences thereof are shown (SEQ ID NO: 5 and SEQ ID NO: 6). In the human genome, they are contained in clone RP11-31F19. They are reported to function as cancer suppressor genes in renal cancer cells (J. Biol. Chem., Vol. 277, Issue 39, 36585-36591, 2002).

### TEK:

TEK is known as CD202B, TIE-2, VMCM (venous malformations, multiple cutaneous and mucosal), VMCM1, or TEK tyrosine kinase, endothelial, and it has the RefSeq ID of NM_000459 (SEQ ID NO: 7). The amino acid sequence thereof is shown (SEQ ID NO: 8). In the human genome, it is contained in clone RP11-33015. It is known as an angiopoietin 1 receptor having tyrosine kinase activity, which is involved in vascular endothelial cell growth (Cell 118: 149-161, 2004).

### EBI2:

EBI2 is known as EBV-induced G-protein coupled receptor 2 or Epstein-Barr virus induced gene 2 (lymphocyte-specific G protein-coupled receptor), and it has the RefSeq ID of NM_004951 (SEQ ID NO: 9). The amino acid sequence thereof is shown (SEQ ID NO: 10). In the human genome, it is contained in clone RP11-72J7. EBI2 is a G-protein-coupled receptor, the ligand of which has not been discovered, and it is known to be induced by infection with EB viruses (J. Virol. 67: 2209-2220, 1993).

Herein, the term "gene" refers to a human-derived gene (genome DNA or cDNA) that is specified with the above nucleotide sequence. The term "protein" refers to a protein that is specified with the above amino acid sequence and encoded by gene.

The GMDS, ANKRD15, TEK, or EBI2 gene may be cDNA obtained from cultured cells using a technique known by persons skilled in the art, or may be synthesized by PCR or the like based on the nucleotide sequences derived from clone information set forth in Table 1 of the present specification. When DNA having the nucleotide sequence set forth in SEQ ID NOs is obtained by PCR, PCR is carried out using a human chromosomal DNA or a cDNA library as a template and a pair of primers designed to be able to amplify the nucleotide sequence derived from clone information set forth in Table 1. DNA fragments amplified by PCR can be cloned into an adequate vector that can be amplified in a host such as *Escherichia coli.*

The aforementioned operations, such as probe or primer preparation, cDNA library construction, screening of a cDNA library, and cloning of a target gene, are known to persons skilled in the art. Such operations can be carried out in accordance with methods described in Molecular Cloning: A laboratory Manual, 2nd Ed., Cold Spring Harbor Laboratory, Cold Spring Harbor, NY, 1989, Current Protocols in Molecular Biology, Supplement 1-38, John Wiley & Sons (1987-1997) and the like.

A homologous gene of GMDS, ANKRD15, TEK, or EBI2 gene may be used. In accordance with the present invention, the term "homologous gene " refers to: a gene having a nucleotide sequence encoding a eaneer-related protein, such nucleotide sequence being derived from the nucleotide sequence derived from clone information set forth in Table 1 by deletion, addition, or substitution of one or several nucleotides; or a gene having a nucleotide sequence encoding a cancer-related protein, such nucleotide sequence being hybridized with the nucleotide sequence derived from clone information set forth in Table 1 under stringent conditions. In addition, a fragment of GMDS, ANKRD15, TEK, or EBI2 gene is included in the definition of homologous gene of GMDS, ANKRD15, TEK, or EBI2 gene.

Regarding the above "nucleotide sequence derived from the nucleotide sequence derived from clone information set forth in Table 1 by deletion, addition, or substitution of one or several nucleotides," the range of "one to several amino acids" is not particularly limited. For instance, such description indicates 1 to 60 nucleotides, preferably 1 to 30 nucleotides, more preferably 1 to 20 nucleotides, further preferably 1 to 10 nucleotides, and particularly preferably 1 to 5 nucleotides,

Thus, as long as the "homologous gene of GMDS, ANKRD15, TEK, or EBI2 gene" has the structure and function described above, its origin is not particularly limited. Therefore, it may be derived from mammals excluding humans, or may be obtained by artificially introducing mutation into a gene derived from mammals such as humans.

The aforementioned "gene having a nucleotide sequence encoding a cancer-related protein, such nucleotide sequence being derived from the nucleotide sequence derived from clone information set forth in Table 1 by deletion, addition, or substitution of one or several nucleotides" can be produced by any methods known by persons skilled in the art such as chemical synthesis, gene engineering techniques, and mutagenesis methods. Specifically, the aforementioned gene can be obtained by utilizing DNA having the nucleotide sequence derived from clone information set forth in Table 1 and introducing mutation into the DNA. For instance, a method wherein DNA having the nucleotide sequence derived from clone information set forth in Table 1 is allowed to come into contact with an agent serving as a mutagen, a method of UV irradiation, a gene engineering technique, and the like can be used. Site-directed mutagenesis is one of gene engineering techniques. It is useful because a specific mutation can be introduced into a specific site. This technique can be carried out in accordance with, for example, Molecular Cloning, A laboratory Manual, 2nd Ed., Cold Spring Harbor Laboratory, Cold Spring Harbor, NY, 1989; Current Protocols in Molecular Biology, Supplements 1-38, John Wiley & Sons (1987-1997).

The aforementioned "nucleotide sequence being hybridized under stringent conditions" refers to a nucleotide sequence of DNA obtained by colony hybridization, plaque hybridization, Southern hybridization, or the like using DNA as a probe. For instance, an example of the DNA used is DNA that can be identified by carrying out hybridization at 65°C in the presence of 0.7 to 1.0 M NaCl using a filter in which DNA derived from a colony or plaque or a fragment thereof is immobilized, followed by washing of the filter at 65°C using 0.1 to 2 x SSC solution (1 x SSC solution comprises 150 mM sodium chloride and 15 mM sodium citrate). Hybridization can be carried out in accordance with methods described in Molecular Cloning: A laboratory Manual, 2nd Ed., Cold Spring Harbor Laboratory, Cold Spring Harbor, NY, 1989.

An example of DNA hybridized under stringent conditions is DNA having a certain level or more of homology to the nucleotide sequence of DNA used as a probe. For instance, such DNA has a homology of 70% or more, preferably 80% or more, more preferably 90% or more, further preferably 93% or more, and particularly preferably 95% or more to the DNA used as a probe.

The aforementioned "gene having a nucleotide sequence encoding a cancer-related protein, such nucleotide sequence being hybridized with the nucleotide sequence derived from clone information set forth in Table 1 under stringent conditions" can be obtained as described above by colony hybridization, plaque hybridization, or Southern hybridization under certain hybridization conditions.

In the present invention, DNA containing part of the GMDS, ANKRD15, TEK, or EBI2 gene may be used to analyze each gene in the test sample. The term "part of the GMDS, ANKRD15, TEK, or EBI2 gene" used herein refers to, for example, an oligonucleotide comprising a nucleotide sequence of about 10 to 30 consecutive nucleotides, among the nucleotide sequences derived from the clone information as shown in Table 1.

The detection for screening cancer can be carried out by analyzing GMDS, ANKRD15, TEK, or EBI2 gene in a test sample using DNA or RNA containing GMDS, ANKRD15, TEK, or EBI2 gene in its entirety or a part thereof as a primer or probe. Specifically, the expression "analyzing GMDS, ANKRD15, TEK, or EBI2 gene" used herein indicates detection of deletion of genome DNA, or detection of gene mutation or abnormal level of gene expression.

Detection of gene mutation is carried out as follows. When the above DNA or RNA is used as a primer, a partial sequence of DNA prepared from a test sample is amplified by PCR using, for example, selected two types of primers

Meanwhile, detection of abnormal levels of gene expression can be carried out by Northern hybridization or RT-PCR (reverse transcription-polymerase chain reaction) using a probe containing the above RNA sequence.

Examples of a test sample that can be used include tissue section, blood, lympha, sputum, lung lavage liquid, urine, feces, and tissue culture supernatant, which are suspected to contain the presence of tumor.

In the present invention, cancer can be diagnosed by analyzing the GMDS, ANKRD15, TEK, or EBI2 proteins in a test sample using an antibody against GMDS, ANKRD15, TEK, or EBI2 protein or a fragment thereof.

The antibody against the GMDS, ANKRD15, TEK, or EBI2 protein used in the present invention (hereafter referred to as the antibody) can be produced by a conventional method using the GMDS, ANKRD15, TEK, or EBI2 protein in its entirety or a part thereof as an antigen. The term "a part of an GMDS, ANKRD15, TEK, OR EBI2 protein" indicates a polypeptide comprising at least 6 amino acids, preferably about 8 to 10 amino acids, and further preferably about 11 to 20 amino acids, which are consecutive amino acids constituting the amino acid sequence of the GMDS, ANKRD15, TEK, OR EBI2 protein set forth in the Sequence Listing. The GMDS, ANKRD15, TEK, OR EBI2 protein in its entirety or a part thereof serving as an antigen may be prepared by either biological or chemical techniques,

A polyclonal antibody can be prepared in the following manner: the above antigen, for example, is repeatedly used for subcutaneous, intramuscular, intraperitoneal, and intravenous inoculation in animals such as mice, guinea pigs, and rabbits such that the animals are sufficiently immunized; blood is collected from the animals; and serum separation is performed. A monoclonal antibody can be prepared from a culture supernatant of a hybridoma or ascites of a mouse into which the hybridoma has been administered, such hybridoma being obtained by cell fusion of commercially available mouse myeloma cells and splenic cells of a mouse immunized with, for example, the above antigen.

It is possible to measure the expression level of GMDS, ANKRD15, TEK, or EBI2 protein in a test sample using an antibody against GMDS, ANKRD15, TEK, or EBI2 protein or a fragment thereof prepared as described above. For instance, the measurement of the expression level can be carried out using Western blotting or immunological methods such as immunoblotting, enzymeimmunoassay (EIA), radioimmunoassay (RIA), a fluorescent antibody method, and immunocyto-staining. Herein, a fragment of GMDS, ANKRD15, TEK, OR EBI2 protein antibody refers to a single-chain fragment variable (scFv) of an antibody of interest or the like.

Examples of a test sample that can be used include tissue section, blood, lympha, sputum, alveolar lavage liquid, urine, feces, and tissue culture supernatant, which are suspected to exhibit the presence of tumor. The low expression level of GMDS, ANKRD15, TEK, or EBI2 protein in a test sample subjected to measurement indicates that the expression of the GMDS, ANKRD15, TEK, or EBI2 gene is suppressed in the sample tissue or cells. Thus, cancer can be diagnosed.

Examples of cancer to be diagnosed in the method for diagnosing cancer according to the present invention include, but are not limited to, malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid gland cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma. Preferably, the cancer to be diagnosed is lung cancer, and particularly preferably non-small cell lung cancer.

The present invention is hereafter described in greater detail with reference to the following examples, but the technical scope of the present invention is not limited thereto.

### Examples

### (1) Experimental materials

Cell lines used herein were squamous cell lines EBC-1, LK-2, PC10, VMRC-LCP, LC-1sq, and ACC-LC-73; adenocarcinoma cell lines 11-18, A549, ABC-1, RERF-LC-OK, VMRC-LCD, SK-LC-3, and RERF-LC-KJ; and large cell carcinoma cell lines KNS-62, 86-2, LU65, PC-13, ACC-LC-33, NCI-H460, and LU99A. As lung cancer samples derived from clinical specimens, paraffin-embedded samples derived from 53 types of adenocarcinoma and rapidly-frozen samples derived from 59 types of adjacent normal site were used. Cancer samples derived from clinical specimens were obtained from the National Cancer Center, Hokushin General Hospital of Nagano Prefectural Welfare Federation and used under agreement with each patient and approval of the ethical committee of each organization. Moreover, clinical specimen donors were not subjected to radiation, chemical therapy, or immunological therapy before sampling.

### (2) Separation of deleted DNA in non-small cell lung cancer cell by array CGH

Non-small cell lung cancer cell lines were screened for a homozygously deleted gene via the array CGH method using an MCG Whole Genome Array-4500 (Inazawa J., et al., Cancer Sci. 95(7), 559, 2004). DNA derived from a cancer cell was labeled with Cy3 and then mixed with a heslthy subject control sample labeled with Cy5, followed by hybridization. BAC clones (each exhibiting a logarithmic value of -2.0 or lower (with 2 as the lowest) obtained by dividing the fluorescent signal intensity of the former DNA by the same of the latter DNA) and the genes contained in such clones are listed in Table 1. Deleted regions were found in various cancer cells; that is, gene deletion represented by cancer-suppressing gene CDKN2 was detected. In the non-small cell lung cancer cell lines, GMDS (6p25: GDP-mannose 4,6-dehydratase), ANKRD15 (9p24.3: ankyin repeat domain 15), TEK (9p21.3: TEK tyrosine kinase, endothelial. TIE2), and EBI2 (13q32.2: Epstein-Barr virus induced gene 2 (lymphocyte-specific G protein-coupled receptor)) were found to be deleted. It was suggested that such deleted gene would function as a cancer-associated gene in the non-small cell lung cancer cell, and it was demonstrated that such deletion could be used as a gene deletion marker for non-small cell lung cancer.

**Table 1: BAC clones separated as homozygously deleted DNAs from non-small cell lung cancer cells via array CGH, and genes contained in such regions**

| No. | MAC | Locus^{a} | | Cell line (Total 20) | | Posable candidate gene^{b} | Number of known genes |
|---|---|---|---|---|---|---|---|
| | | Chr. Band | Position | n | Name | | |
| 1 | RP11-175M15 | 1p30.21 | chr1:13,731,635-13,731,866 | 1 | 11-16 | *PRDM2* | 2 |
| | | | | | | | |
| 2 | RP11-78M24 | 8p25 | chrs:1,754,013-1,926,781 | 1 | KNS-52 | *GMDS* | 1 |
| | | | | | | | |
| 3 | RP11-31F19 | 8p24.3 | chrs:537,217-682,143 | 1 | LC-1 sq | *ANKRO15* | 7 |
| | RP11-143M15 | 8p24.3 | ch9:812,146-991,182 | 1 | LC-1 sq | | |
| | | | | | | | |
| 4 | RP11.113D19 | 8p21.3 | chr9:20,996,400-21,158,464 | 1 | VMRC-LCD | *CDKN2A, CDKN2B, MTAP* | 22 |
| | RP11-344A7 | 8p21.8 | chr9:21,505,373-21,676,227 | 4 | VMRC-LCD, LC-1 eq, KNS-52 | | |
| | RP11-11J1 | Sp21.3 | chr8:22.417.726-22.579,721 | 1 | KNS-52 | | |
| | RP11-782K2 | Sp21.3 | chr8:22,584,881-22,585,358 | 1 | KN8-62 | | |
| | | | | | | | |
| 5 | RP11-33O15 | Sp21.3 | chr8:823,087-22,897,484 | 2 | KN8-62, LU98A | TEX | 0 |
| | RP11-330J23 | 8p21.3 | chr8:25,292,187-25,425,885 | 2 | KN8-62, LU9A | | |
| | RP11-55P8 | 8p21.3-21.2 | chr8:25,425,787-25,573,598 | 1 | LU99A | | |
| | | | | | | | |
| 6 | RP11-3070D17 | 13q2.2 | chr13:56.727,654-61,388,564 | 1 | VMRC-LCD | PCDH20 | 2 |
| | | | | | | | |
| 7 | RP11-600P1 | 13q31.1 | chr13:77,973,420-78,588,321 | 1 | VMRC-LCP | | 4 |
| | RP11-25J23 | 13q31,1 | chr13:78,637,347-78,310,431 | 1 | VMRC-LCP | | |
| | RP11-440G4 | 13q31,1 | chr13:81,026,555-81,027,175 | 1 | VMRC-LCP | | |
| | RP11-295L12 | 13q31.1 | chr13:81,571,893-62,147,460 | 1 | VMRC-LCP | | |
| | RP110400MB | 13q31.1 | chr13:82,201,212-52,280,653 | 1 | VMRC-LCP | | |
| | RP11-91O15 | 13q31.1 | chr13:63,558,326-83,670,185 | 1 | VMRC-LCP | | |
| | RP11-118K20 | 13q31.1 | chr13:83,711,618-83,853,235 | 1 | VMRC-LCP | | |
| | RP11-29C8 | 13q31.1 | chr13:84,853,778-85,313,978 | 1 | VMRC-LCP | | |
| | RP11-589120 | 13q31.1 | chr13:85,691,468-88,093,882 | 1 | VMRC-LCP | | |
| | RP11-29P20 | 13q31.2 | chr13:86,796,851-87,270,500 | 1 | VMRC-LCP | | |
| | RP11-27D9 | 13q31.2 | chr13:87,913,237-88,370,388 | 1 | VMRC-LCP | | |
| | RP11-114O1 | 13q31.3 | chr13:88,858,527-88,117.305 | 1 | VMRC-LCP | | |
| | RP11-86C8 | 13q31.3 | chr13.88,883,889-89,379,575 | 1 | VMRC-LCP | | |
| | RP11-78H7 | 13q31.3 | chr13:88,556,353-90,080,636 | 1 | VMRC-LCP | | |
| | | | | | | | |
| 8 | RP11-72J7 | 13q32.2 | chr13:97,465,157-97,495,769 | 1 | VMRC-LCD | *EB12* | 10 |
| | RP11-19J14 | 13q32.2 | chr13:97,851,595-97,851,757 | 1 | VMRC-LCD | | |
| | RP11-122A8 | 13q32.3 | chr13:96,471,182-98,644,517 | 1 | VMRC-LCD | | |
| | | | | | | | |
| 9 | RP11-134O22 | 20p12.1 | chr20;15,224,211-15,251,444 | 2 | SK-LC-3, KNS-62 | | 0 |
| | RP11-65O18 | 20p12.1 | chr20:15,271,770-15,375,903 | 1 | SK-LC-3 | | |
| | RP11-11O15 | 20p12.1 | chr20:15,587,853-15,726,758 | 1 | SK-LG-3 | | |

| | | | | | | | |
|---|---|---|---|---|---|---|---|
| "Based on UCSC Genome Browner, May 2004 Assembly, "Possible tumor suppressor genes located around BAC. | | | | | | | |

### (3) Conclusions

By screening using array CGH, a gene that is homozygously deleted in its genomic DNA, i.e., a gene that lacks genomic region in the non-small cell lung cancer cell line, was isolated. By detecting the existence of such gene, cancer can be diagnosed.

### Effects of the Invention

The present invention provides a novel method for diagnosing cancer comprising detecting the cancer-associated GMDS, ANKRD15, TEK, or EBI2 gene or the GMDS, ANKRD15, TEIC, or EBI2 protein encoded by such gene. Analysis of such gene or gene product is very useful from the clinical point of view in terms of improvement in treatment or cancer prognosis based on individuality of cancer or from the viewpoint of fundamental cancer research. Also, assay of the mRNA expression level of the GMDS, ANKRD15, TEK, or EBI2 gene enables selection of a patient with non-small cell lung cancer.

## Claims

1. A method for diagnosing cancer which comprises a step of detecting deletion of the GMDS, ANKRD15, TEK, or EBI2 gene in a test sample by using DNA containing all or part of said gene.

2. A method for diagnosing cancer which comprises a step of analyzing the GMDS, ANKRD15, TEK, or EBI2 gene in a test sample by using DNA or RNA containing all or part of the GMDS, ANKRD15, TEK, or EBI2 gene.

3. The method of claim 2 wherein the analysis involves detection of mutation of the gene or detection of abnormal expression level of the gene.

4. A method for diagnosing cancer which comprises a step of analyzing GMDS, ANKRD15, TEK, or EBI2 protein in a test sample using an antibody against GMDS, ANKRD15, TEK, OR EBI2 protein or fragment thereof.

5. The method of claim 4 wherein the analysis involves detection of abnormal expression level of the protein.

6. The method of claim 1 wherein lung cancer is diagnosed.

7. The method of claim 2 wherein lung cancer is diagnosed.

8. The method of claim 4 wherein lung cancer is diagnosed.
